(19) Europäisches Patentamt / European Patent Office / Office européen des brevets

(11) **EP 3 299 305 A1**

(12) **EUROPEAN PATENT APPLICATION**
published in accordance with Art. 153(4) EPC

(43) Date of publication:
**28.03.2018 Bulletin 2018/13**

(21) Application number: **16796461.8**

(22) Date of filing: **16.05.2016**

(51) Int Cl.:
**B65B 55/16** (2006.01)   **A23L 3/28** (2006.01)
**A61L 2/10** (2006.01)

(86) International application number:
**PCT/JP2016/064437**

(87) International publication number:
**WO 2016/186068 (24.11.2016 Gazette 2016/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.05.2015 JP 2015101459**

(71) Applicant: **Tokuyama Corporation**
**Shunan-shi, Yamaguchi 745-8648 (JP)**

(72) Inventor: **MATSUI, Shingo**
**Shunan-shi**
**Yamaguchi 745-8648 (JP)**

(74) Representative: **Markfort, Iris-Anne Lucie**
**Lorenz & Kollegen**
**Patentanwälte Partnerschaftsgesellschaft mbB**
**Alte Ulmer Straße 2**
**89522 Heidenheim (DE)**

(54) **ULTRAVIOLET STERILIZATION METHOD, METHOD FOR MANUFACTURING CONTAINER-PACKAGED PRODUCT, AND ULTRAVIOLET STERILIZATION DEVICE**

(57)    Provided is a sterilization method capable of performing ultraviolet sterilization with high reliability on, for example, an object to be sterilized having a low ultraviolet transmittance, such as foods and beverages, capable of substantially eliminating a cleaning operation for an ultraviolet transmissive window material of an ultraviolet irradiation apparatus, and having low risk of recontamination.

In the sterilization method, for example, when an ultraviolet transmissive container such as a flexible bag made of an ultraviolet transmissive film of a heat-sealing resin is filled with an object to be sterilized and is then irradiated with ultraviolet light from the outside of the container to perform sterilization on the object to be sterilized, the container filled with the object to be sterilized is air-tightly sealed during a period from the end of the filling to the end of the ultraviolet light irradiation, or the ultraviolet light irradiation and the sealing are performed under an identical sterile environment.

FIG.1

## Description

Technical Field

The present invention relates to a sterilization method using ultraviolet light.

Background Art

In contrast to sterilization using a medical agent, ultraviolet sterilization generates few residues, has high safety, and hardly changes an object to be irradiated. Therefore, the ultraviolet sterilization is suitable as a sterilization method for foods or medical products for which security and safety are expected. It is thus proposed that the ultraviolet sterilization is used in sterilization for various situations.

For example, Patent Literatures 1 to 4 respectively describe the following ultraviolet sterilization apparatuses. Specifically, Patent Literature 1 describes an ultraviolet sterilization apparatus that includes: a flow path surrounded by a material having transmission properties to ultraviolet light having a sterilization action, through which a fluid object to be sterilized passes; and a light source that is disposed outside the flow path and emits ultraviolet light having a sterilization action, in which sterilization is performed by irradiating the object to be sterilized, which passes through the flow path, with the ultraviolet light emitted from the light source.

Patent Literature 2 describes a wastewater treatment apparatus that treats organic substances in wastewater. The wastewater treatment apparatus includes: a wastewater treatment tank that stores wastewater; a light source that applies ultraviolet light; at least one photocatalyst covered light leaking and introducing body that is installed in the wastewater treatment tank and is formed by covering the surface of a light leaking and introducing body with a photocatalyst layer containing a photocatalyst, the light leaking and introducing body including a light introducing unit and a light leaking unit, the light introducing unit introducing the light applied from the light source, the light leaking unit having an optical refraction index that is equal to or larger than that of the light introducing unit and leaking ultraviolet light from the light introducing unit; a light introducing body that introduces the ultraviolet light applied from the light source to the photocatalyst covered light leaking and introducing body; and an oxygen supply unit that is provided in the wastewater treatment tank and supplies oxygen toward the photocatalyst covered light leaking and introducing body. The wastewater treatment apparatus decomposes and removes the organic substances in the wastewater by the photocatalyst and ozone ($O_3$) generated by the oxygen supplied from the oxygen supply unit.

Patent Literature 3 describes an ultraviolet irradiation water treatment apparatus including an LED module disposed in a treatment tank, the LED module being constituted by mutually combining the back surfaces of first and second heat transfer plates, which include a plurality of ultraviolet LEDs disposed on the surfaces thereof and are respectively equipped with protective covers covering the ultraviolet LEDs in a watertight state, in a state where the ultraviolet LED of the first heat transfer plate and the ultraviolet LED of the second heat transfer plate are not overlapped back to back.

Patent Literature 4 describes an ultraviolet sterilization apparatus includes: a flow path surrounded by a material having transmission properties to deep ultraviolet light with a wavelength of 200 to 350 nm having a sterilization action, through which a fluid object to be sterilized passes; and a light source that is disposed outside the flow path and emits the deep ultraviolet light having a sterilization action, in which sterilization is performed by irradiating the object to be sterilized, which passes through the flow path, with the deep ultraviolet light emitted from the light source. The light source includes an ultraviolet light-emitting element disposing base and a cover. In the ultraviolet light-emitting element disposing base, a plurality of "ultraviolet light-emitting elements that emit deep ultraviolet light" are disposed on a side surface of a cylindrical or polygonal columnar base such that the optical axis of each ultraviolet light-emitting element passes through the center axis of the cylindrical or polygonal columnar base, and the deep ultraviolet light is radially emitted to the center axis. The cover is made of a deep ultraviolet transmissive material. The cover is airtightly mounted to the ultraviolet light-emitting element disposing base so as to cover the ultraviolet light-emitting element disposing base and seal inert gas or dry air therein. The cover is constituted of a deep ultraviolet light-emitting module that includes a flow path for cooling medium, which is formed in the cylindrical or polygonal columnar base and through which a cooling medium passes. The ultraviolet sterilization apparatus includes a light condensing and deep ultraviolet emitting unit that includes the light source disposed on a focal axis of a long elliptical reflective mirror or a parabolic reflective mirror, and condenses and emits the deep ultraviolet light radially emitted from the light source, in which the object to be sterilized is irradiated with the condensed deep ultraviolet light emitted from the light condensing and deep ultraviolet emitting unit.

Further, Patent Literature 5 describes a surface-emitting device that includes a light source and a light guide plate including the light source disposed on a side surface, in which at least one of the front surface and the back surface of the light guide plate is a light-emitting surface radiating light from the light source, surfaces of the light guide plate other than the light-emitting surface and the side surface on which the light source is disposed are formed as light-blocking surfaces, and light having a peak wavelength of 388 nm or less is radiated.

Citation List

Patent Literature

Patent Literature 1: WO 2010/058607
Patent Literature 2: Japanese Patent No. 5566801
Patent Literature 3: Japanese Patent Application Laid-open No. 2012-115715
Patent Literature 4: Japanese Patent Application Laid-open No. 2014-87544
Patent Literature 5: Japanese Patent Application Laid-open No. 2006-237563

Disclosure of Invention

Technical Problem

It is known that ultraviolet transmittance varies depending on the type of substance. For example, in pure water, the ultraviolet transmittance is relatively high, whereas in an aqueous solution in which a solute that absorbs ultraviolet light is dissolved or in a suspension in which suspended matters that absorb or scatter ultraviolet light are included, the ultraviolet transmittance decreases. The rate of decrease significantly changes depending on the types of solutes or suspended matters or on a contained amount thereof. Specifically, it is known that in distilled water, a thickness (light path length: length at which light passes through a sample) at which the transmittance to ultraviolet light with 253.7 nm is 10% is 300 mm, whereas in milk and juice, the thicknesses are 0.07 mm and 0.5 to 1 mm, respectively. Therefore, assuming that ultraviolet sterilization is performed on a solution or a suspension by the conventional technologies described in Patent Literatures 1 to 4, there is a concern that sterilization is insufficiently performed.

Further, in the inventions described in Patent Literatures 1 to 4, an object to be sterilized is irradiated with ultraviolet light with the object to be sterilized being in contact with an "ultraviolet transmissive window material" attached to an ultraviolet irradiation apparatus (or ultraviolet sterilization apparatus) at a site from which ultraviolet light is emitted. Thus, a solute component, suspended matters, and the like of the object to be sterilized adhere to the window material. This needs cleaning of the window material by a major method such as regular water washing or disassembling and cleaning.

Furthermore, in those conventional technologies, even in a case where the sterilization is sufficiently performed by ultraviolet irradiation, there is a risk that microorganism is mixed again when the object to be sterilized is filled into a container or the like or is retained in a tank after the sterilization is performed.

In this regard, it is an object of the present invention to provide a sterilization method capable of performing ultraviolet sterilization with high reliability on liquid objects to be sterilized, e.g., beverages such as juice and milk, and liquid drugs such as drops, and also on objects to be sterilized having a low ultraviolet transmittance, e.g., paste-like, jelly-like, and mousse-like objects to be sterilized such as nursing care foods and desserts, capable of substantially eliminating a cleaning operation for an ultraviolet transmissive window material of an ultraviolet irradiation apparatus, and having low risk of recontamination.

Solution to Problem

According to an embodiment of the present invention, there is provided an ultraviolet sterilization method including: a filling step of filling an ultraviolet transmissive container with an object to be sterilized; an ultraviolet light irradiation step of irradiating the container with ultraviolet light from the outside of the container, the container being filled with the object to be sterilized by the filling step; and a sealing step of air-tightly sealing the container filled with the object to be sterilized, in which the sealing step is performed during a period from an end of the filling step to an end of the ultraviolet light irradiation step, or the ultraviolet light irradiation step and the sealing step are performed under an identical sterile environment.

In the ultraviolet sterilization method, on the grounds that recontamination can be prevented without fail, it is favorable that the ultraviolet transmissive container is a flexible bag made of an ultraviolet transmissive film of a heat-sealing resin or a flexible bag made of an ultraviolet-transmissive laminated resin film including a heat-sealing resin layer, and the sealing step is performed by heat-sealing of an aperture of the flexible bag.

Further, in the ultraviolet sterilization method, it is favorable that in the ultraviolet light irradiation step, "a maximum length at which an optical axis of the ultraviolet light applied crosses a container" or "intensity of ultraviolet light to be applied" in the ultraviolet transmissive container is controlled such that irradiance of the ultraviolet light at an arbitrary position of the object to be sterilized, which is filled into the container, is set to 0.01 mW/cm$^2$ or more, favorably 0.03 mW/cm$^2$ or more, and most favorably 0.05 mW/cm$^2$ or more.

Further, in the ultraviolet sterilization method, from the viewpoint of preservation stability of the object to be sterilized, it is favorable to further include an ultraviolet blocking step of performing ultraviolet blocking treatment on an outer surface of the ultraviolet transmissive container sealed by the sealing step.

In the ultraviolet sterilization method, because of having no standby time, using no mercury, easy maintenance, long life span, and the like, one including an ultraviolet light-emitting diode (UV-LED) is favorably used as the ultraviolet light source. In particular, a deep ultraviolet light-emitting diode (DUV-LED) having a main peak of an emission wavelength in the wavelength range of 200 nm to 300 nm, particularly in the wavelength range of 220 nm to 280 nm, is favorably used.

Further, according to an embodiment of the present invention, there is provided a method of manufacturing a container-packaged product in which food, a cosmetic, a quasi-drug, or a drug, which is subjected to ultraviolet sterilization, is sealed in a container, the method including a step of filling a container with a fluid object to be sterilized including food, a cosmetic, a quasi-drug, or a drug, performing ultraviolet sterilization, and sealing the container by the ultraviolet sterilization method described above.

Furthermore, according to an embodiment of the present invention, there is provided an ultraviolet sterilization apparatus including a pair of partition walls and ultraviolet light sources.

The pair of partition walls each include an ultraviolet transmissive window and are configured to be capable of pinching an ultraviolet transmissive container with the ultraviolet transmissive windows, the ultraviolet transmissive container being filled with an object to be sterilized.

The ultraviolet light sources are each provided to the inside of the pair of partition walls and irradiate the ultraviolet transmissive container with ultraviolet light via the ultraviolet transmissive windows.

The ultraviolet transmissive window may have a shape corresponding to a shape of the pinched ultraviolet transmissive container.

The ultraviolet light source typically includes a plurality of linear or point light sources disposed along the light-emitting surface.

Alternatively, the pair of partition walls may each include an aperture closed by the ultraviolet transmissive window, and the ultraviolet light source may be configured to be movable inside the aperture along the ultraviolet transmissive window.

Advantageous Effects of Invention

In the ultraviolet sterilization method of the present invention, the object to be sterilized is filled into the ultraviolet transmissive container and is sterilized by irradiating the container with ultraviolet light from the outside of the container. Thus, the object to be sterilized does not come into contact with the window material of the ultraviolet irradiation apparatus or the ultraviolet sterilization apparatus, and the window material is not contaminated. Therefore, there is no need to perform disassembling and cleaning, and a clean state (highly ultraviolet-transmissive state) can be constantly kept by an easy operation such as an operation of wiping light dirt like attached dust.

Further, in the ultraviolet sterilization method of the present invention, the sealing step is performed during a period from the end of the filling step to the end of the ultraviolet light irradiation step, or the ultraviolet light irradiation step and the sealing step are performed under an identical sterile environment. Thus, recontamination after the ultraviolet sterilization can be prevented.

Furthermore, in the ultraviolet sterilization method of the present invention, in a case where an object to be sterilized that is freely deformable depending on deformation of a container, such as a liquid, paste-like, jelly-like, or mousse-like object to be sterilized, is used and where a flexible bag made of an ultraviolet transmissive film of a heat-sealing resin or a flexible bag made of an ultraviolet-transmissive laminated resin film including a heat-sealing resin layer is used as a container, the container can be deformed to make the width thereof smaller, and the ultraviolet irradiation can be performed such that the ultraviolet light can easily reach the inside sufficiently. Thus, the reliability of sterilization can be enhanced.

Moreover, in the method of the present invention, in a case where an ultraviolet blocking step of performing ultraviolet blocking treatment on an outer surface of the ultraviolet transmissive container sealed by the sealing step is added, when the sterilized object, which is sealed in the ultraviolet transmissive container, is preserved for a long period of time, it is possible to prevent change in quality of the content (sterilized object) or deterioration of the container due to ultraviolet light from nature.

In so-called retort foods, it is necessary to seal food in a retort pouch and then perform pressurizing and heating sterilization. On the other hand, in the method of the present invention, it is unnecessary to perform pressurizing and heating. Thus, objects to be sterilized on which pressurizing and heating sterilization cannot be performed, e.g., fresh foods such as raw milk and fermented milk, pure soy sauce, pure sake, and draft beer, can also be sterilized.

Further, according to the method of manufacturing a container-packaged product in the present invention, in addition to the effects obtained by the ultraviolet sterilization method of the present invention as described above, filling of a container with foods, cosmetics, quasi-drugs, or drugs having fluidity, ultraviolet sterilization, and sealing of the container can be performed. Besides, compared with the case where the pressurizing and heating sterilization is performed, advantages such as simplicity of the apparatus, low energy cost, and reduced time for manufacturing can be provided.

Brief Description of Drawings

[Fig. 1] Fig. 1 is a transverse cross-sectional view of an ultraviolet sterilization apparatus that can be suitably used in an ultraviolet light irradiation step of a method according to an embodiment of the present invention.

[Fig. 2] Fig. 2 is a transverse cross-sectional view and vertical cross-sectional view of an ultraviolet light-emitting module that can be suitably used in the ultraviolet sterilization apparatus shown in Fig. 1.

[Fig. 3] Fig. 3 is a schematic view for describing a suitable mode of an ultraviolet light irradiation step of a method according to an embodiment of the present invention.

[Fig. 4] Fig. 4 is an outline view of an ultraviolet light irradiation unit suitably used in the mode shown in Fig. 3.

[Fig. 5] Fig. 5 is an outline view of another ultraviolet light irradiation unit suitably used in the mode shown in Fig. 3.

[Fig. 6] Fig. 6 is an outline view of still another ultraviolet light irradiation unit suitably used in the mode shown in Fig. 3.

[Fig. 7] Fig. 7 is a transverse cross-sectional view of a light source (modularized light source for light condensing) in the ultraviolet light irradiation unit shown in Fig. 4.

[Fig. 8] Fig. 8 is a side view of the light source (modularized light source for light condensing) in the ultraviolet light irradiation unit shown in Fig. 4.

Mode(s) for Carrying Out the Invention

According to an embodiment of the present invention, there is provided an ultraviolet sterilization method including: a filling step of filling an ultraviolet transmissive container with an object to be sterilized; an ultraviolet light irradiation step of irradiating the container with ultraviolet light from the outside of the container, the container being filled with the object to be sterilized by the filling step; and a sealing step of air-tightly sealing the container filled with the object to be sterilized, in which the sealing step is performed during a period from an end of the filling step to an end of the ultraviolet light irradiation step, or the ultraviolet light irradiation step and the sealing step are performed under an identical sterile environment.

An object to be sterilized in this embodiment is not particularly limited as long as the object can be filled into a container. However, because ultraviolet sterilization of this embodiment exerts a noticeable effect, the object to be sterilized is favorably an object having fluidity, in which the thickness at which transmittance to ultraviolet light of 253.7 nm is 10% (light path length: length at which light passes through a sample) is 100 mm or less and 0.001 mm or more. In particular, a liquid, paste-like, jelly-like, or mousse-like object to be sterilized in which the thickness is 10 mm or less and 0.001 mm is particularly favorable. Examples of such an object to be sterilized include foods (including beverages), cosmetics, quasi-drugs, and drugs having fluidity. Specifically, examples of the foods include liquid foods, liquid seasoning, edible oil, alcohol, beverages, yogurt, ice cream, and jelly. Examples of the cosmetics include various skin cosmetics such as cosmetic liquid, face lotion, cream, milk lotion, and facial cleanser, makeup products such as foundation and makeup base, perfume, and eau de cologne. Further, examples of the quasi-drugs include energy drinks, toothpaste, and hair care products, and examples of the drugs include eye drops, various drops, various injection medicines, and various ointments. Of those, in objects unsuitable for pressurizing and heating sterilization, e.g., objects including active ingredients decomposed or changed in quality by pressure heating, such as raw milk, fresh juice, pure sake, draft beer, and pure soy sauce, an effect of this embodiment is found particularly noticeable. Thus, using those objects as objects to be sterilized is particularly favorable.

An ultraviolet transmissive container used in this embodiment is not particularly limited as long as the container is made of an ultraviolet transmissive material. Further, the ultraviolet transmissive container only needs to have a part made of an ultraviolet transmissive material, the part coming into contact with an object to be sterilized when the object to be sterilized is filled thereinto. The ultraviolet transmissive container is not necessarily made of the ultraviolet transmissive material as a whole. It is favorable to use an ultraviolet transmissive resin as the ultraviolet transmissive material, from the viewpoint of easiness of manufacturing of the container.

The ultraviolet transmissive resin is favorably a resin having transmission properties to deep ultraviolet light with the wavelength of 200 nm to 300 nm, particularly 220 nm to 280 nm. Examples of such a resin include a polyolefin resin or polyolefin copolymer resin such as polyethylene, polypropylene, and polymethylterpene, a fluorine resin such as polytetrafluoroethylene and tetrafluoroethylene/perfluoro(alkyl vinyl ether) copolymer, a methacrylic resin, an epoxy resin, an alicyclic polyimide resin, a polyamide resin, polyvinyl chloride, and a polyvinyl alcohol resin (any of which favorably contains no additives such as an ultraviolet absorber that absorbs applied ultraviolet light in the ultraviolet light irradiation step and a plasticizer). Of those resins, the polyolefin resin or polyolefin copolymer resin having high heat-sealing properties is favorably used. Those ultraviolet transmissive resins may be used independently or may be used in combination like a laminated body. It should be noted that data regarding ultraviolet transmittance of a resin film is described in: Etsuzo MATSUI and Yoshihiro SHIMIZU, "Ultraviolet Transmittance of Plastic Film II", Report of Toyo Junior College of Food Technology and Toyo Institute of Food Technology, 102-111 (1967); Daikin Industries, Ltd., Technical Material GX-27e, "NEOFLON$_{TM}$ film"; http://jp.mitsuichem.com/service/functional_polymeric/polyme rs/tpx/spec.htm; and the like.

The shape of the ultraviolet transmissive container is not particularly limited. The ultraviolet transmissive container may be any of a bag-like container, a box-like container, and a bottle-like container or may have other shapes. It should be noted that, from the viewpoint of ultraviolet transmission properties, the thickness of a container material (the thickness of a partition wall, sheet, or film that partitions the outside and the inside of the container) is favorably thin as long as it has strength capable of holding content. The thickness of the container material is favorably 5 $\mu$m or more and 1 mm or less, more favorably 10 $\mu$m or more and 500 $\mu$m or less, and particularly favorably 20 $\mu$m or more and 300 $\mu$m or less. Further, the transmittance of the container material (partition wall, sheet, or film that partitions the outside and the inside thereof) to ultraviolet light to be irradiated is favorably 50% or more, more favorably 70% or more, and most favorably 75% or more.

Of those containers made of the ultraviolet transmissive resin, from the viewpoint of enabling heat-sealing in the sealing step, the ultraviolet transmissive resin container is favorably a flexible bag made of an ultraviolet transmissive film of a heat-sealing resin or a flexible bag made of an ultraviolet-transmissive laminated resin film including a heat-sealing resin layer. It should be noted that examples of the ultraviolet-transmissive laminated resin film including a heat-sealing resin layer include one as disclosed in Japanese Patent Application Laid-open No. 2013-534874.

The form of the flexible bag described above is not limited as long as the flexible bag is a flexible bag made of an ultraviolet transmissive film of a heat-sealing resin or a flexible bag made of an ultraviolet-transmissive laminated resin film including a heat-sealing resin layer and has an aperture from which an object to be sterilized is filled. Well-known forms such as a two-sided bag, a three-sided bag, a gusset bag, a bottom gusset bag, a stand up bag, a side-seal bag, and a bottom-seal bag can be employed. Those bags may include spouts, zippers, or fasteners.

The filling step is not particularly different from a conventional filling step except for use of the ultraviolet transmissive resin container as a container, and can be performed using, for example, an automatic pouch filling machine or an automatic bottle filling machine.

The sealing step is performed by air-tightly sealing the container. As a sealing method, heat-sealing or bonding with use of an adhesive can be suitably employed for a bag-like container, and capping can be suitably employed for a bottle-shaped container. In sealing, gas replacement treatment or deaeration treatment can also be performed. Those treatment can be easily performed using a dedicated machine generally used in so-called vacuum packaging or gas filling packaging.

In the ultraviolet sterilization method of this embodiment, the sealing step is performed during a period from the end of the filling step to the end of the ultraviolet light irradiation step (detailed thereof will be described later), or the ultraviolet light irradiation step and the sealing step are performed under an identical sterile environment. Here, performing under an identical sterile environment means that the ultraviolet light irradiation step and the sealing step are performed in a space where a sterile state is kept, such as an indoor space that is, for example, sufficiently sterilized and to which sterilized air is supplied at a positive pressure, without taking out a container filled with an object to be sterilized to the outside of the space. At that time, the sealing step can also be performed after ultraviolet irradiation.

In the ultraviolet light irradiation step, the container filled with the object to be sterilized is irradiated with ultraviolet light from the outside thereof. At that time, the ultraviolet light to be applied is favorably deep ultraviolet light having the wavelength of 200 nm to 300 nm, and particularly 220 nm to 280 nm. Further, although an ultraviolet lamp such as a mercury lamp can be used as an ultraviolet light source, in application to an industrial manufacturing process, it is favorable to use an ultraviolet light-emitting diode (UV-LED), and particularly a deep ultraviolet light-emitting diode (DUV-LED) that emits deep ultraviolet light having the favorable wavelength described above. This is because the UV-LED (or DUV-LED) has advantages such as using no mercury, having no standby time, low power consumption, and high durability, unlike a mercury lamp. A DUV-LED having a main peak wavelength in the range of 200 nm to 300 nm, and particularly in the range of 220 nm to 280 nm, can be suitably used. Use of ultraviolet light having the wavelength in the range of 220 nm to 280 nm enables sterilization of an object to be sterilized without generating ozone or the like.

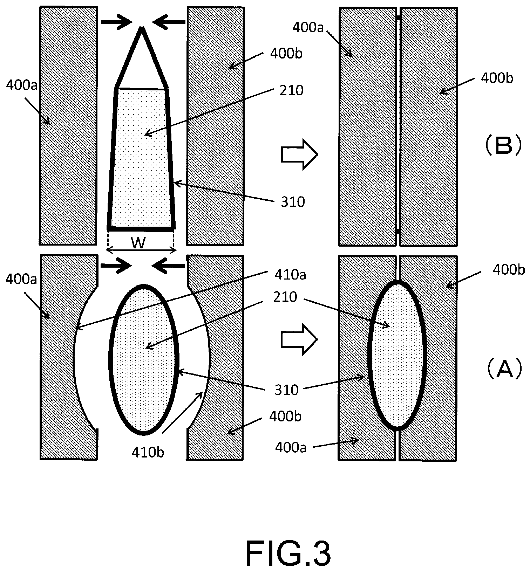

In the ultraviolet light irradiation step, a method of irradiating the container filled with the object to be sterilized with ultraviolet light from the outside thereof is not particularly limited. However, in a case where an object to be sterilized having a low ultraviolet transmittance is used, irradiation with high-intensity ultraviolet light is favorably performed. As described above, in an object to be sterilized including a solution or a suspension, the irradiation intensity of the ultraviolet light rapidly attenuates as the length of the object to be sterilized in a thickness direction becomes larger (as the depth becomes larger), and the ultraviolet light does not reach the object to be sterilized inside. Therefore, in order to cause the ultraviolet light to reach the inside, it is necessary to make the width of the container (maximum length at which the optical axis of the ultraviolet light to be applied crosses the container, e.g., W in Fig. 3) smaller (thinner) or make the intensity of the ultraviolet light to be applied stronger. In the method of this embodiment, it is favorable to control the width of the container or the intensity of the ultraviolet light such that the irradiance of the ultraviolet light at an arbitrary position of the object to be sterilized, which is filled into the container, is set to 0.01 mW/cm$^2$ or more, particularly favorably 0.03 mW/cm$^2$ or more, and most favorably 0.05 mW/cm$^2$ or more. A higher irradiance is better. Although setting the upper limit of the irradiance does not have a special meaning, the irradiance is generally 1 W/cm$^2$ or less.

It should be noted that the value of the irradiance, 0.01 mW/cm$^2$, 0.03 mW/cm$^2$, or 0.05 mW/cm$^2$, does not have a significance of critical range in the numerical value itself and is an indicator determined from the viewpoint that a sterilization effect can be effectively obtained in an industrially-practical treatment time (ultraviolet irradiation time). For example, assuming sterilization of Escherichia coli, in which an ultraviolet irradiation amount (cumulative irradiation amount) necessary for 99.9% of deactivation is approximately 10 $mJ/cm^2$ (= $mW \cdot sec/cm^2$), at the irradiance of 0.01 $mW/cm^2$, 0.03 $mW/cm^2$, and 0.05 $mW/cm^2$, 99.9% of deactivation is enabled by irradiation for 1,000 seconds (16 minutes and 40 seconds), 333 seconds (5 minutes and 33 seconds), and 200 seconds (3 minutes and 20 seconds), respectively. For comparison, in retort foods, pressurizing and heating sterilization is performed approximately at the center temperature of 120$\underline{o}$C for 4 minutes or more.

In order to meet such conditions, the following operations are favorably performed for a case (i) of ultraviolet light irradiation from one direction and a case (ii) of ultraviolet light irradiation from two directions facing each other on an identical optical axis. Specifically, assuming that the transmittance of a partition wall, sheet, or film, which partitions the outside and the inside of a container, to ultraviolet light to be applied is T(%), in the case (i) of ultraviolet light irradiation from one direction, the irradiance of ultraviolet light that has passed through a container filled with liquid to be sterilized only needs to be set to 0.01 $(mW/cm^2) \times T/100 = T \times 10^{-4}$ $(mW/cm^2)$ or more, favorably $3T \times 10^{-4}$ $(mW/cm^2)$ or more, and most favorably $5T \times 10^{-4}$ $(mW/cm^2)$ or more. Further, in the case (ii) of ultraviolet light irradiation from two directions facing each other on an identical optical axis, the irradiance of ultraviolet light at the center within the container is favorably set to 0.01 $mW/cm^2$ or more, more favorably 0.03 $mW/cm^2$ or more, and most favorably 0.05 $mW/cm^2$ or more.

For the object to be sterilized actually used, a relationship between the light path length and the irradiance of the transmitted ultraviolet light is investigated in advance by, for example, a procedure as shown in the following steps (a) to (d). Thus, in the cases (i) and (ii), the irradiance at each position can be easily estimated.

The relationship between the irradiance of the transmitted ultraviolet light and the light path length in the steps (a) to (d) (S101 to S104) follows the Lambert-Beer law:

(a) Step S101 of filling an ultraviolet-transmissive cell for optical measurement having a predetermined light path length (hereinafter, simply referred to as "cell" in some cases) with an object to be sterilized;

(b) Step S102 of bringing an ultraviolet light-emitting surface of an ultraviolet irradiation apparatus into close contact with the cell and applying ultraviolet light toward the inside of the cell, the ultraviolet light being emitted from the ultraviolet light-emitting surface under light emission conditions that are identical to those in sterilization treatment;

(c) Step S15 of measuring the irradiance of transmitted ultraviolet light that has passed through the cell (unit: $mW/cm^2$) ; and

(d) Step S104 of performing the above Steps (a) to (c) (S101 to S103) on a plurality of cells having different light path lengths, to calculate a relationship between the irradiance of the transmitted ultraviolet light and the light path length. In other words, an irradiance $I_1$ of the transmitted ultraviolet light has a relationship of the following Expression (1) to a light path length L.

$$\log(I_1/I_0) = -\alpha L \ \dots \ (1)$$

In Expression (1), $I_0$ represents the irradiance of ultraviolet light having a wavelength $\lambda$ before entering a medium, and $\alpha$ represents a proportional constant (absorption coefficient) determined to correspond to the object to be sterilized and a wavelength $\lambda_{peak}$. In general, a peak width of an emission spectrum of a light-emitting diode is extremely narrow. Thus, in discussion of the light path length dependence on the irradiance of transmitted ultraviolet light, it suffices to take into consideration only an absorption coefficient $\alpha$ ($\lambda_{peak}$) at an emission peak wavelength $\lambda_{peak}$ of a deep ultraviolet light-emitting diode. Expression (1) can be modified as the following Expression (2).

$$\log I_1 = -\alpha L + \log I_0 \ \dots \ (2)$$

Therefore, when a plurality of sets of a logarithm of an irradiance $I_1$ of the transmitted ultraviolet light having a main peak wavelength $\lambda_{peak}$ and the light path length L of the cell are obtained, it is possible to calculate a regression line that associates the irradiance $I_1$ of the transmitted ultraviolet light having the main peak wavelength $\lambda_{peak}$ with the light path length L (Step S104 of the above (d)). In order to calculate the regression line, a well-known method such as a least-squares method can be used.

It should be noted that in order to easily estimate the irradiance at each position in the above cases (i) and (ii), on the basis of the relationship between the light path length and the irradiance of the transmitted ultraviolet light, which is determined as described above, it is necessary to correct the irradiance $I_1$ by using an ultraviolet transmittance T (%) of the container material. This is because the ultraviolet light passes through the container material (a partition wall, sheet, or film that partitions the outside and the inside of the container) and is applied to the object to be sterilized.

Such a method probably enables estimation with significantly high accuracy, but it is favorable to ultimately perform determination under actual use conditions in a real machine. If the conditions are stable, results do not change. Thus, such determination is unnecessary to be performed every time. In normal cases, the determination only needs to be performed when the apparatus is activated and when the conditions are changed. Further, in a case where the apparatus is continuously operated for a long period of time, the determination only needs to be performed regularly in each certain period of time.

In control of the width of the container or the intensity of the ultraviolet light, in a case where a thin container cannot be used and where a container is not deformed, the intensity of ultraviolet light to be applied only needs to be controlled. In a case where a thin container can be used or a container is deformable like a flexible bag (a container can be deformed and its width can be controlled), the width of the container and/or the intensity of the ultraviolet light only need to be controlled.

The ultraviolet light-emitting diode (UV-LED), particularly the deep ultraviolet light-emitting diode (DUV-LED) has weaker light-emitting output and higher directivity of emitted ultraviolet light than a ultraviolet lamp, and thus an irradiation region of the ultraviolet light is narrowed. Therefore, in a case where the width of the container is particularly difficult to control and where the UV-LED or DUV-LED is used as a light source, it is necessary to irradiate the ultraviolet transmissive container with high-intensity ultraviolet light over the entire surface to be irradiated with ultraviolet light.

For a method of irradiating the ultraviolet transmissive container with high-intensity ultraviolet light over the entire surface to be irradiated with ultraviolet light, a method of increasing the irradiance by using light condensing or a method of increasing the light-emitting output by providing a high current in a forward direction with use of a step-up DC-DC converter or a charge pump (at that time, light pulses may be emitted as needed) is suitably employed.

For example, in a case where the ultraviolet transmissive container is an axisymmetric hollow body like a bottle and is difficult to deform (which is temporarily deformed by an action of a force thereon and is restored to the original shape by relaxing the force or causing a restoring force to act), the following method can be suitably employed.

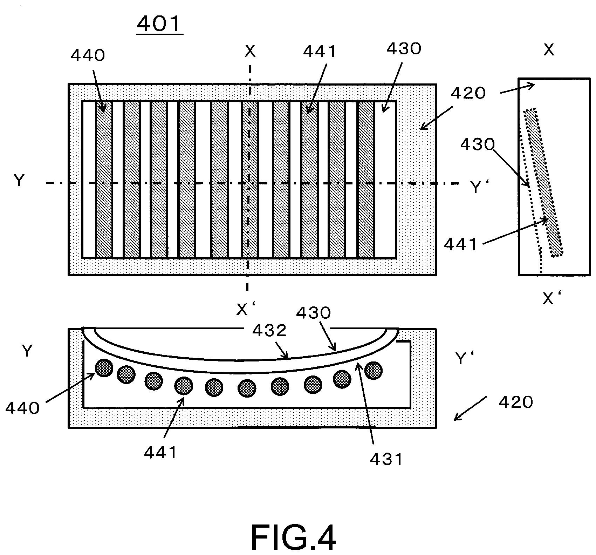

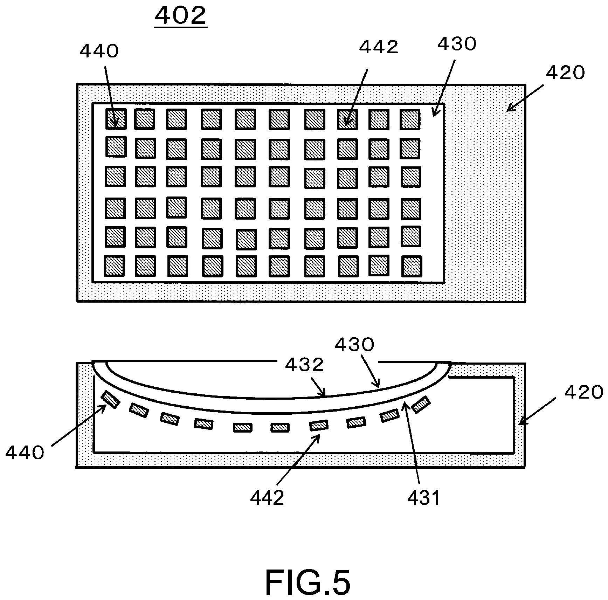

In other words, first, a method (also referred to as method 1) of performing ultraviolet irradiation by disposing a container filled with an object to be sterilized, instead of a flow path of an "ultraviolet sterilization apparatus" (at a position where the flow path is disposed) as shown in Fig. 4 or 5 of Patent Literature 4 can be suitably employed. The ultraviolet sterilization apparatus includes: a flow path surrounded by a material having transmission properties to ultraviolet light having a sterilization action, through which a fluid object to be sterilized passes; and a light source that is disposed outside the flow path and emits ultraviolet light having a sterilization action, in which sterilization is performed by irradiating the object to be sterilized, which passes through the flow path, with the ultraviolet light emitted from the light source. The light source includes a plurality of "ultraviolet light-emitting elements that emit ultraviolet light having a sterilization action". The ultraviolet sterilization apparatus further includes a light condenser that condenses the ultraviolet light emitted from each of the ultraviolet light-emitting elements, the object to be sterilized being irradiated with the ultraviolet light condensed by the light condenser.

Second, a method (also referred to as method 2) of arraying many DUV-LEDs over the entire inner surface of a cylindrical base material capable of housing a container, the inner surface facing the container, and applying ultraviolet light with high output by providing a high current in a forward direction with use of a step-up DC-DC converter or a charge pump can be suitably employed.

Further, in a case where the shape of the ultraviolet transmissive container can be freely set, a thin (for example, plate-like) container may be used, and the intensity of ultraviolet light may be increased as needed. For example, in a case where the ultraviolet transmissive container is a flexible bag, the bag is favorably formed into a thin elliptical columnar shape (including a shape with thickness gradually decreasing in the height direction) or a quadratic prism (or plate-like) shape. Further, the ultraviolet irradiation is favorably performed by the following method (also referred to as method 3). Specifically, in the method 3, the ultraviolet transmissive container filled with an object to be sterilized is disposed in a space between a pair of partition walls disposed to face each other with a predetermined width interval being provided therebetween such that a surface of the ultraviolet transmissive container comes close to or abuts on the partition wall, and is irradiated with ultraviolet light from one or two ultraviolet light-emitting surfaces provided to one or both of the two facing surfaces of the pair of partition walls. At that time, in a case where the surface of only one of the partition walls is set as the ultraviolet light-emitting surface, the surface of the other partition wall (the surface facing the ultraviolet light-emitting surface) is favorably set as an ultraviolet light reflective mirror.

Furthermore, in the method 3 described above, the following method (also referred to as method 3-1) is favorable: disposing the ultraviolet transmissive container filled with the object to be sterilized such that 50% or more of an area of the surface thereof comes close to or abuts on the partition wall, and irradiating the ultraviolet transmissive container with ultraviolet light from the two ultraviolet light-emitting surfaces provided to both of the two facing surfaces of the pair of partition walls. In particular, the following method (also referred to as method 3-2) is favorably employed: forming the ultraviolet light-emitting surface into a shape corresponding to the shape of the surface of the ultraviolet transmissive container filled with the object to be sterilized such that 80% or more of an area of the surface of the ultraviolet transmissive container comes close to or abuts on the partition wall, and performing ultraviolet irradiation.

Further, in a case where the object to be sterilized is liquid, paste-like, jelly-like, or mousse-like and where the ultraviolet transmissive container is a flexible bag made of an ultraviolet transmissive film of a heat-sealing resin or a flexible bag made of an ultraviolet-transmissive laminated resin film including a heat-sealing resin layer, the following method (also referred to as method 3-3) is favorably employed: pinching the flexible bag filled with the object to be sterilized from its front side and/or a back side by the ultraviolet light-emitting surfaces, deforming the flexible bag such that the thickness thereof is made smaller, and performing the ultraviolet irradiation.

Hereinafter, the methods 1 and 3-3 described above will be described in detail with reference to the drawings.

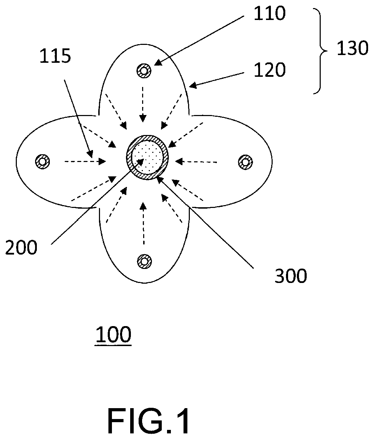

Fig. 1 is a transverse cross-sectional view of an ultraviolet sterilization apparatus 100 used in the method 1. This apparatus is basically the same apparatus as that shown in Fig. 4 of Patent Literature 4, in which a bottle-like ultraviolet transmissive container 300 filled with an object to be sterilized 200 is disposed at a position where a quartz tube or sapphire tube forming the flow path of the apparatus shown in Fig. 4 is disposed. The ultraviolet sterilization apparatus 100 shown in Fig. 1 includes a "light condensing and ultraviolet emitting unit" 130 including: a light source including an ultraviolet light-emitting module 110 where a plurality of deep ultraviolet light-emitting elements are disposed on a side surface of a cylindrical base such that an optical axis of each deep ultraviolet light-emitting element passes through the center axis of the base and the deep ultraviolet light is radially emitted to the center axis; and a light condenser including a long elliptical reflective mirror 120, the ultraviolet light-emitting module 110 being disposed on a focal axis of the long elliptical reflective mirror 120 to condense and emit the ultraviolet light radially emitted from the ultraviolet light-emitting module 110. In the ultraviolet sterilization apparatus 100, the four light condensing and ultraviolet emitting units 130 are disposed such that respective light condensing axes thereof coincide with one another, and the ultraviolet transmissive container 300 is disposed such that those light condensing axes coincide with a symmetry axis (center axis) of the bottle-like ultraviolet transmissive container 300.

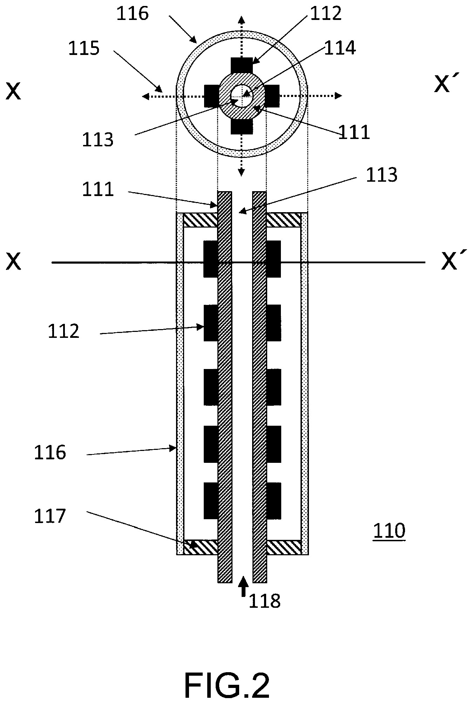

As shown in Fig. 2, the ultraviolet light-emitting module 110 includes a plurality of DUV-LEDs 112 arrayed on a surface of a cylindrical base 111, and a flow path for cooling medium 113 is formed inside the cylindrical base. Further, the cylindrical base 111 equipped with the DUV-LEDs 112 is covered with a cover 116 formed of an ultraviolet transmissive material such as quartz or sapphire. The cover 116 is air-tightly or water-tightly mounted to the cylindrical base by using a sealing member 117 such as a sealant, a packing, or an o-ring. The inside thereof is filled with an inert gas such as nitrogen or gas such as dry air.

The DUV-LEDs are disposed in a state where the elements are mounted on a sub mount or are housed in a package and such that ultraviolet light is emitted in a certain direction. It should be noted that although not shown in the figure, wiring for supplying electric power to the deep ultraviolet light-emitting elements from the outside, circuits for operating correctly the DUV-LEDs, and the like are formed on the sub mount or the package. Electrical power is supplied to the wiring or the circuits via wiring formed on the surface of the cylindrical base 111 or inside the cylindrical base 111.

The plurality of DUV-LEDs 112 are disposed on the side surface of the cylindrical base 111 along a circumferential direction of the base such that an optical axis 115 of each DUV-LED 112 passes through a center axis 114 of the base 111. As a result, deep ultraviolet light emitted from the DUV-LEDs are radially emitted to the center axis 114.

As described above, since the ultraviolet transmissive container 300 is disposed such that the symmetry axis (center axis) thereof coincides with each of the light condensing axes of the four light condensing and ultraviolet emitting units 130, all the deep ultraviolet light radially emitted from the respective ultraviolet light-emitting modules 110 are condensed so as to converge to the symmetry axis (center axis) of the ultraviolet transmissive container 300. In such a manner, in the ultraviolet sterilization apparatus 100, in principle, all the deep ultraviolet light radially emitted from the ultraviolet light-emitting modules 110 can be applied to the ultraviolet transmissive container 300, and ultraviolet light emitted to a direction other than a direction toward the ultraviolet transmissive container 300 (for example, the opposite direction or horizontal direction) can also be efficiently used. Consequently, high-intensity ultraviolet light can be applied.

Fig. 3 a schematic view for describing the method 3-3, in which the lower part (A) is a bottom view and the upper part (B) is a front view. In the method 3-3, a liquid, paste-like, jelly-like, or mousse-like one is used as an object to be sterilized 210, and a flexible bag made of an ultraviolet transmissive film of a heat-sealing resin or a flexible "stand up bag having an elliptical bottom surface" made of an ultraviolet-transmissive laminated resin film including a heat-sealing resin layer is used as an ultraviolet transmissive container 310. First, in a filling step and a sealing step that are not shown in the figure, the ultraviolet transmissive container 310 is filled with the object to be sterilized 210 and is further sealed. Subsequently, as shown in Fig. 3, when the ultraviolet transmissive container 310 is disposed at the center of a space between ultraviolet light irradiation units 400a and 400b, which are a pair of partition walls disposed to face each other with a predetermined width interval being provided therebetween, the ultraviolet light irradiation units 400a and 400b automatically move in a direction toward the ultraviolet transmissive container 310, so that the ultraviolet transmissive container 310 is pinched by ultraviolet light-emitting surfaces 410a and 410b of the respective ultraviolet light irradiation units. The ultraviolet transmissive container 310 is then deformed such that the thickness thereof is made small. The ultraviolet irradiation is thus performed.

The ultraviolet light irradiation units 400a and 400b have a basically identical structure except for mutually having a mirror image relationship, and the light-emitting surfaces 410a and 410b thereof have a shape corresponding to the shape of the pinched ultraviolet transmissive container 310. The light-emitting surfaces 410a and 410b of the ultraviolet light irradiation units used in Fig. 3 are each a concave curve that is inclined to correspond to the shape of the stand up bag (ultraviolet transmissive container 310) having an elliptical bottom surface. The shape of the light-emitting surface may be appropriately changed depending on the shape of the ultraviolet transmissive container. For example, in a case where the ultraviolet transmissive container is a flat bag (plate-like ultraviolet transmissive container), the shape is made flat.

Figs. 4 to 6 respectively show ultraviolet light irradiation units 401, 402, and 403 suitably used in the method shown in Fig. 3. Each of those ultraviolet light irradiation units includes a casing 420 having an aperture, an ultraviolet transmissive window 430 that passes ultraviolet light therethrough, and an ultraviolet light source 440. The ultraviolet transmissive window 430 has an inner side surface 431 and an outer side surface 432 on the opposite side of the inner side surface, and the inner side surface is disposed to face the inside of the casing so as to close the aperture of the casing. Each of those ultraviolet light irradiation units irradiates an object to be sterilized (filled into the ultraviolet transmissive container), which is disposed to face the outer side surface 432 of the ultraviolet transmissive window, with ultraviolet light and thus performs sterilization. Therefore, the outer side surface 432 of the ultraviolet transmissive window 430 becomes the light-emitting surface 410a (410b) of Fig. 3.

A material of the casing 420 is not particularly limited as long as it does not pass ultraviolet light. For example, metal, resin, and the like can be employed. It should be noted that the inner surface of the casing 420, more specifically, a surface of a part that can be seen from the outside of the ultraviolet transmissive window 430 is favorably formed of an ultraviolet reflective material. Examples of the ultraviolet reflective material that can be suitably used in this embodiment include chromium (ultraviolet reflectance: approximately 50%), platinum (ultraviolet reflectance: approximately 50%), rhodium (ultraviolet reflectance: approximately 65%), barium sulfate (ultraviolet reflectance: approximately 95%), magnesium carbonate (ultraviolet reflectance: approximately 75%), calcium carbonate (ultraviolet reflectance: approximately 75%), magnesium oxide (ultraviolet reflectance: approximately 90%), and aluminum (ultraviolet reflectance: approximately 90%). Of those, rhodium, platinum, or aluminum is particularly favorably used as the ultraviolet reflective material, because high reflectance can be provided to the surface by surface treatment such as a plating method or a vapor deposition method. It should be noted that in a case where a metal material is employed as the ultraviolet reflective material, from the viewpoint of prevention of decrease in reflectance because of oxidation or damage of the surface, the surface of the ultraviolet reflective material is favorably covered with the ultraviolet transmissive material such as quartz, sapphire, or a polytetrafluoroethylene film.

The ultraviolet transmissive window 430 has the inner side surface 431 and the outer side surface 432 on the opposite side of the inner side surface, and the inner side surface is provided to face the inside of the casing 420 so as to close the aperture of the casing 420. The ultraviolet transmissive window 430 passes ultraviolet light emitted from the light source 440 through the ultraviolet transmissive window 430 and the partition wall (or sheet or film) of the ultraviolet transmissive container 310 and irradiates the object to be sterilized 210 with the ultraviolet light. For example, sapphire, quartz, or the like can be favorably employed as a material of the ultraviolet transmissive window 430. Additionally, the ultraviolet transmissive window 430 can be suitably formed of a molded body or flexible sheet (or film) made of an ultraviolet transmissive resin. Examples of such an ultraviolet transmissive resin include polytetrafluoroethylene, polyethylene, polypropylene, a methacrylic resin, an epoxy resin, an alicyclic polyimide resin, a polyamide resin, polyvinyl chloride, and a polyvinyl alcohol resin, which favorably contain no additives such as an ultraviolet absorber that absorbs applied ultraviolet light and a plasticizer.

It should be noted that in a case where the ultraviolet transmissive window is formed of the ultraviolet transmissive resin, the resin may be deteriorated by ultraviolet irradiation. Thus, the ultraviolet transmissive window is favorably attached with a detachable structure like a casing, from the viewpoint of facilitating replacement of the ultraviolet transmissive window.

In the ultraviolet light irradiation units 401, 402, and 403 shown in Figs. 4 to 6, as described above, the ultraviolet transmissive window 430 has a concave curve that is inclined to correspond to the shape of a stand up bag (ultraviolet transmissive container 310).

The light source 440 differs between the ultraviolet light irradiation units 401, 402, and 403. In the ultraviolet light irradiation unit 401 shown in Fig. 4, a plurality of ultraviolet lamps 441 (linear light sources) are used as the light source. The ultraviolet lamps 441 are arranged at predetermined intervals along the inner side surface 431 of the ultraviolet transmissive window 430. An ultraviolet light reflective mirror (not shown in the figure) is installed at the back of the lamps. Ultraviolet light emitted backward from the ultraviolet lamps 441 is reflected on the mirror and travels toward the ultraviolet lamps 441 from clearances between the lamps.

In the ultraviolet light irradiation unit 402 shown in Fig. 5, a plurality of DUV-LEDs 442 (point light sources) are used as the light source. The plurality of DUV-LEDs 442 are mounted on a substrate (not shown in the figure) mainly made of metal having high thermal conductivity, such as copper or aluminum, ceramics, or the like, and are arrayed so as to face the inner side surface 431 of the ultraviolet transmissive window 430. The DUV-LEDs 442 are usually packaged or modularized and emit light with increased directivity, such as parallel light. However, it is favorable that light is radially emitted with a certain emission angle such that a light intensity distribution is made more uniform. The number of DUV-LEDs 442 may be the number enough to irradiate the entire inner side surface 431 of the ultraviolet transmissive window 430 with the emitted ultraviolet light. It should be noted that as the number of DUV-LEDs 442 increases, the intensity of ultraviolet light becomes stronger. Further, similarly to the method 2, high output can be achieved by providing a high current in a forward direction with use of a step-up DC-DC converter or a charge pump.

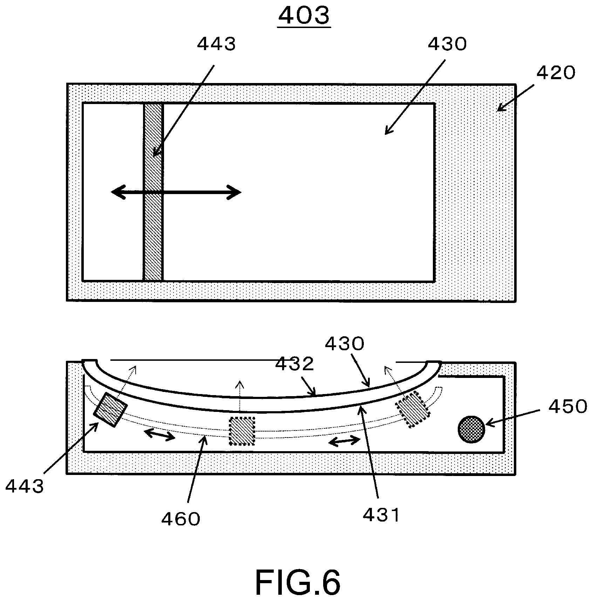

In the ultraviolet light irradiation unit 403 shown in Fig. 6, a light source (hereinafter, also referred to as "modularized light source for light condensing") 443 is used as the light source. The light source 443, which incorporates the ultraviolet light-emitting module 110 shown in Fig. 2, condenses ultraviolet light radially emitted to the center axis 114 of the ultraviolet light-emitting module 110 and emits the ultraviolet light as a belt-like light flux. The length of the ultraviolet light-emitting module 110 is set to be matched with the width of the ultraviolet transmissive window 430 (the length of a narrow side). Thus, when the modularized light source for light condensing 443 is disposed within the casing 420 and caused to slide along the ultraviolet transmissive window 430, the entire inner side surface 431 of the ultraviolet transmissive window 430 can be irradiated with the ultraviolet light by scanning of the belt-like light flux.

The modularized light source for light condensing 443 is described as an ultraviolet light-emitting apparatus in Japanese Patent No. 5591305, the content of which is incorporated herein with reference thereto.

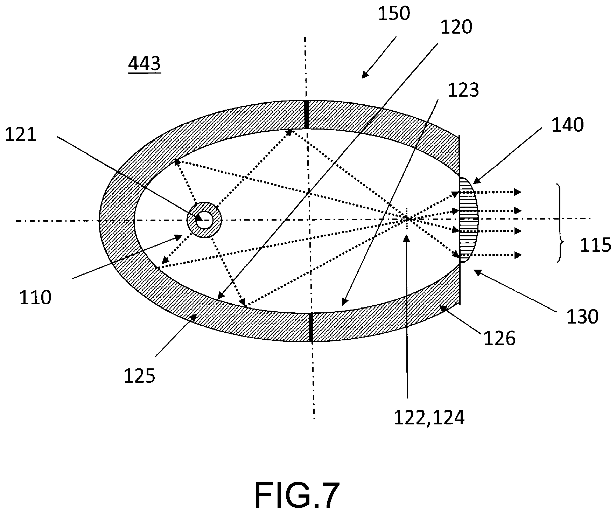

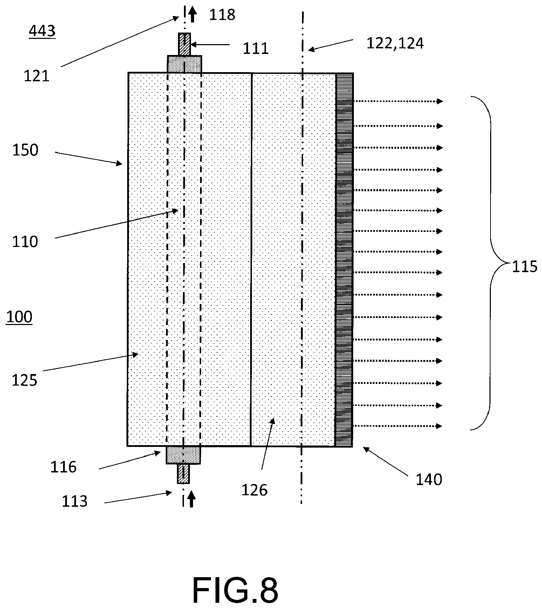

Figs. 7 and 8 show a transverse cross-sectional view and a side view of the modularized light source for light condensing 443 including a rod-like light source 110. The modularized light source for light condensing 443 includes a main body 150 including a light-emission-side casing 125, a light-condensing-side casing 126, and a collimating optical system 140. The light-emission-side casing 125 has an inner surface that is a light-emission-side reflective mirror 120 constituted of a long elliptical reflective mirror. The light-condensing-side casing 126 has an inner surface that is a light-condensing-side reflective mirror 123 constituted of a long elliptical reflective mirror, and also includes an aperture for emitting deep ultraviolet light 130. The collimating optical system 140 is disposed at the aperture for emitting deep ultraviolet light 130. The rod-like light source 110 is disposed inside the main body 150. In the main body 150, it is favorable that the light-emission-side casing 125 and the light-condensing-side casing casing 126 are mutually attachable/detachable or openable/closeable using a hinge or the like. Further, the top and bottom apertures on both ends of the main body 150 shown in Figs. 7 and 8 are provided with covers (not shown in the figure) for preventing ultraviolet light from leaking to the outside.

In the mode shown in Figs. 7 and 8, the light-emission-side reflective mirror 120 and the light-condensing-side reflective mirror 123 are the long elliptical reflective mirrors having substantially the same shapes. Thus, an inner space formed by binding the light-emission-side casing 125 and the light-condensing-side casing 126 in the main body 150 has a columnar shape having an elliptical cross-section (it should be noted that a part corresponding to the aperture 130 is missing) with two focal axes, i.e., a focal axis 121 of the light-emission-side reflective mirror and a light condensing axis 122 of the light-emission-side reflective mirror. Surfaces of the light-emission-side reflective mirror 120 and the light-condensing-side reflective mirror 123 are favorably formed of a material having a large reflectance to deep ultraviolet light, e.g., a platinum group metal such as Ru, Rh, Pd, Os, Ir, and Pt; Al; Ag; Ti; an alloy containing at least one of these metals; or magnesium oxide. Because of especially high reflectance, it is particularly favorable that those surfaces are formed of Al, a platinum group metal, an alloy containing the platinum group metal, or magnesium oxide.

The light-condensing-side reflective mirror 123 and the light-condensing-side casing 126 are provided with the aperture for emitting deep ultraviolet light 130 in a slit manner. At the aperture 130, the collimating optical system 140 for converting ultraviolet light condensed into a parallel or substantially parallel light flux is disposed. The collimating optical system 140 is favorably formed of a material having high ultraviolet transmission properties, such as synthesized or natural quartz, sapphire, and an ultraviolet transmissive resin. The collimating optical system 140 is favorably attached to the aperture for emitting deep ultraviolet light 130 in a detachable manner.

In the modularized light source for light condensing 443, the rod-like light source 110 is disposed such that the center axis 114 thereof coincides with the focal axis 121 of the light-emission-side reflective mirror. Since the rod-like light source 110 is disposed at such a position, the deep ultraviolet light emitted radially from the rod-like light source 110 is reflected on the light-emission-side reflective mirror 120 and the light-condensing-side reflective mirror 123 and is condensed to converge on a focal axis 124 of the light-condensing-side reflective mirror (i.e., light condensing axis 122 of the light-emission-side reflective mirror). The condensed deep ultraviolet light is emitted toward the inner side surface 431 of the ultraviolet transmissive window 430.

In such a manner, in the modularized light source for light condensing 443, in principal, all the deep ultraviolet light radially emitted from the rod-like light source 110 can be condensed on the focal axis 124 of the light-condensing-side reflective mirror 123, and ultraviolet light emitted to a direction other than a direction toward the aperture for emitting deep ultraviolet light 130 (for example, the opposite direction or horizontal direction) can also be efficiently used. In other words, there is no need to dispose all the deep ultraviolet LEDs 112, 112, ... on a coplanar surface such that the optical axes 115 are directed to the aperture for emitting deep ultraviolet light 130 in the rod-like light source 110. The deep ultraviolet LEDs 112, 112, ... can also be disposed in the horizontal direction or the opposite direction. Therefore, in the rod-like light source 110, the number of ultraviolet light-emitting elements disposed per unit space can be greatly increased. In the modularized light source for light condensing 443, higher-intensity ultraviolet light can be emitted. Further, in the modularized light source for light condensing 443, there is no need to use a field lens having a large diameter. Furthermore, in the modularized light source for light condensing 443, ultraviolet light is emitted as a belt-like light flux, and an irradiation region can be irradiated with ultraviolet light having uniform intensity not in a narrow spot-like manner but in a rectangular form with a long side.

In the ultraviolet light irradiation unit 403, the modularized light source for light condensing 443 is disposed within the casing 420 so as to emit the belt-like light flux toward the inner side surface 431 of the ultraviolet transmissive window 430. Further, an electric motor 450 and a pair of guide rails 460 are disposed within the casing 420. The modularized light source for light condensing 443 is held by the guide rails 460 and driven by the electric motor 450, to reciprocate (slide) on the guide rails in directions of the arrows in the lower part of Fig. 6. For a mechanism (not shown in the figure) for converting a rotary drive force of the electric motor 450 into a drive force of linear motion along the guide rails 460, well-known rotational motion-linear motion conversion mechanisms such as a rack and pinion mechanism, a crank mechanism, a cam mechanism, and a belt mechanism can be employed without particular limitation. The modularized light source for light condensing 443 performs such reciprocating motion (sliding) and thus performs scanning with the ultraviolet light, so that the entire inner side surface 431 of the ultraviolet transmissive window 430 can be irradiated with the ultraviolet light.

Each of the ultraviolet light irradiation units 401 to 403 may also include a heat-sealing unit that seals the ultraviolet transmissive container 310. The heat-sealing unit includes, for example, a heater disposed within the casing, and a pinching unit that pinches a sealing portion of the ultraviolet transmissive container. When the pinching unit is heated with the heater, a sealing step can be performed before, during, or after the ultraviolet irradiation on the object to be sterilized.

Furthermore, the ultraviolet light irradiation units 401 to 403 described above are configured to irradiate the single ultraviolet transmissive container 310 with ultraviolet light, but the ultraviolet transmissive container may be formed by coupling a plurality of ultraviolet transmissive containers. In this case, when the ultraviolet light irradiation unit is also formed by coupling a plurality of ultraviolet light irradiation units, the objects to be sterilized in the plurality of ultraviolet transmissive containers can be simultaneously subjected to sterilization treatment.

The embodiment described above has described the ultraviolet light irradiation unit with use of an example where the ultraviolet transmissive container is a stand up bag with an elliptical bottom surface, but the light condensing and ultraviolet emitting unit is not limited thereto. For example, as described above, the shape of the light-emitting surface may be appropriately changed depending on the shape of the ultraviolet transmissive container. For example, in a case where the ultraviolet transmissive container is flat bag (plate-like ultraviolet transmissive container), the shape is made flat. Further, the surface-emitting device as disclosed in Patent Literature 5 can also be used as the ultraviolet light irradiation unit.

An object obtained by the ultraviolet sterilization method of this embodiment, which is obtained when an object to be sterilized is subjected to ultraviolet sterilization and sealed in an ultraviolet transmissive container, can be distributed as it is as a commercial product such as a container-packaged product (for example, container-packaged foods, container-packaged cosmetics, container-packaged quasi-drugs, or container-packaged drugs). However, in order to prevent change in quality of the object to be sterilized or deterioration of the container due to ultraviolet light from nature at the time of preservation or during the course of distribution, it is favorable to further include an ultraviolet blocking step of performing ultraviolet blocking treatment on the outer surface of the ultraviolet transmissive container sealed by the sealing step.

A method of performing the ultraviolet blocking treatment on the outer surface of the ultraviolet transmissive container only needs to cover a portion of the container with an ultraviolet non-transmissive material, the portion passing ultraviolet light therethrough. Examples of a covering method include a method of performing printing using an ultraviolet non-transmissive ink, a method of performing surface coating with an ultraviolet non-transmissive coating agent, a method of attaching (laminating) an ultraviolet non-transmissive film.

The ultraviolet sterilization method of this embodiment is suitably applicable to a method of manufacturing a container-packaged product where foods, cosmetics, quasi-drugs, or drugs having fluidity, which are subjected to ultraviolet sterilization, are sealed in a container.

Reference Signs List

100 ultraviolet sterilization apparatus
110 ultraviolet light-emitting module

111 cylindrical base

112, 442 deep ultraviolet light-emitting element (DUV-LED)

113 flow path for cooling medium

114 center axis of cylindrical base

115 optical axis of deep ultraviolet light-emitting element

116 cover

117 sealing member

118 cooling medium

120 light-emission-side reflective mirror constituted of long elliptical reflective mirror

121 focal axis of light-emission-side reflective mirror

122 light condensing axis of light-emission-side reflective mirror

123 light-condensing-side reflective mirror constituted of long elliptical reflective mirror

124 focal axis of light-condensing-side reflective mirror

125 light-emission-side casing

126 light-condensing-side casing

130 light condensing and ultraviolet emitting unit (combination of 110 and 120)

140 collimating optical system

150 main body

200, 210 object to be sterilized

300, 310 ultraviolet transmissive container

400a, 400b, 401, 402, 403 ultraviolet light irradiation unit

420 casing

430 ultraviolet transmissive window

431 inner side surface

432 outer side surface

440 light source

441 ultraviolet lamp

443 modularized light source for light condensing

450 electric motor

460 guide rail

**Claims**

1. An ultraviolet sterilization method, comprising:

   a filling step of filling an ultraviolet transmissive container with an object to be sterilized;
   an ultraviolet light irradiation step of irradiating the container with ultraviolet light from the outside of the container, the container being filled with the object to be sterilized by the filling step; and
   a sealing step of air-tightly sealing the container filled with the object to be sterilized, wherein
   the sealing step is performed during a period from an end of the filling step to an end of the ultraviolet light irradiation step, or the ultraviolet light irradiation step and the sealing step are performed under an identical sterile environment.

2. The ultraviolet sterilization method according to claim 1, wherein
   the ultraviolet transmissive container is a flexible bag made of an ultraviolet transmissive film of a heat-sealing resin or a flexible bag made of an ultraviolet-transmissive laminated resin film including a heat-sealing resin layer, and
   the sealing step is performed by heat-sealing of an aperture of the flexible bag.

3. The ultraviolet sterilization method according to claim 1 or 2, wherein
   in the ultraviolet light irradiation step, "a maximum length at which an optical axis of the ultraviolet light applied crosses a container" or "intensity of ultraviolet light to be applied" in the ultraviolet transmissive container is controlled such that irradiance of the ultraviolet light at an arbitrary position of the object to be sterilized, which is filled into the container, is set to 0.01 mW/cm$^2$ or more.

4. The ultraviolet sterilization method according to any one of claims 1 to 3, wherein
   in the ultraviolet light irradiation step,
   the ultraviolet transmissive container filled with the object to be sterilized is disposed in a space between a pair of partition walls disposed to face each other with a predetermined width interval being provided therebetween such that a surface of the ultraviolet transmissive container comes close to or abuts on the partition wall, and is irradiated with ultraviolet light from one or two ultraviolet light-emitting surfaces provided to one or both of two facing surfaces of the pair of partition walls.

5. The ultraviolet sterilization method according to claim 4, wherein
   the ultraviolet transmissive container filled with the object to be sterilized is disposed such that 50% or more of an area of the surface of the ultraviolet transmissive container comes close to or abuts on the partition wall, and is irradiated with ultraviolet light from the two ultraviolet light-emitting surfaces respectively provided to both of the two facing surfaces of the pair of partition walls.

6. The ultraviolet sterilization method according to claim 5, wherein
   the ultraviolet light-emitting surface is formed into a shape corresponding to a shape of the surface of the ultraviolet transmissive container filled with the object to be sterilized, and ultraviolet irradiation is performed such that 80% or more of an area of the surface of the ultraviolet transmissive container comes close to or abuts on the partition wall.

7. The ultraviolet sterilization method according to any one of claims 4 to 6, wherein

the object to be sterilized is liquid, paste-like, jelly-like, or mousse-like,
the ultraviolet transmissive container is a flexible bag made of an ultraviolet transmissive film of a heat-sealing resin or a flexible bag made of an ultraviolet-transmissive laminated resin film including a heat-sealing resin layer, and
the flexible bag filled with the object to be sterilized is pinched from a front side and/or a back side thereof by the ultraviolet light-emitting surface and is deformed such that the thickness thereof is made smaller, to perform ultraviolet irradiation.

8. The ultraviolet sterilization method according to any one of claims 1 to 7, further comprising
an ultraviolet blocking step of performing ultraviolet blocking treatment on an outer surface of the ultraviolet transmissive container sealed by the sealing step.

9. A method of manufacturing a container-packaged product in which food, a cosmetic, a quasi-drug, or a drug, which is subjected to ultraviolet sterilization, is sealed in a container, the method comprising
a step of filling a container with a fluid object to be sterilized including food, a cosmetic, a quasi-drug, or a drug, performing ultraviolet sterilization, and sealing the container by the ultraviolet sterilization method according to any one of claims 1 to 8.

10. An ultraviolet sterilization apparatus, comprising:

a pair of partition walls that each include an ultraviolet transmissive window and are configured to be capable of pinching an ultraviolet transmissive container with the ultraviolet transmissive windows, the ultraviolet transmissive container being filled with an object to be sterilized; and
ultraviolet light sources that are each provided to the inside of the pair of partition walls and irradiate the ultraviolet transmissive container with ultraviolet light via the ultraviolet transmissive windows.

11. The ultraviolet sterilization apparatus according to claim 10, wherein
the ultraviolet transmissive window has a shape corresponding to a shape of the pinched ultraviolet transmissive container.

12. The ultraviolet sterilization apparatus according to claim 10 or 11, wherein
the ultraviolet light source includes a plurality of linear or point light sources disposed along the light-emitting surface.

13. The ultraviolet sterilization apparatus according to any one of claims 10 to 12, wherein
the pair of partition walls each include an aperture closed by the ultraviolet transmissive window, and the ultraviolet light source is configured to be movable inside the aperture along the ultraviolet transmissive window.

**Amended claims under Art. 19.1 PCT**

1. (Currently amended) An ultraviolet sterilization method, comprising:

a filling step of filling an ultraviolet transmissive container with a liquid, paste-like, jelly-like, or mousse-like object to be sterilized, the ultraviolet transmissive container being a flexible bag made of an ultraviolet transmissive film of a heat-sealing resin or a flexible bag made of an ultraviolet-transmissive laminated resin film including a heat-sealing resin layer;
an ultraviolet light irradiation step of irradiating the container with ultraviolet light from the outside of the container, the container being filled with the object to be sterilized by the filling step; and
a sealing step of air-tightly sealing the container filled with the object to be sterilized, wherein
in the ultraviolet light irradiation step, the ultraviolet transmissive container filled with the object to be sterilized is disposed in a space between a pair of partition walls disposed to face each other with a predetermined width interval being provided therebetween such that a surface of the ultraviolet transmissive container filled with the object to be sterilized comes close to or abuts on the partition wall, is pinched from a front side and/or a back side thereof by the ultraviolet light-emitting surface and deformed such that the thickness thereof is made smaller, and is irradiated with ultraviolet light from one or two ultraviolet light-emitting surfaces provided to one or both of two facing surfaces of the pair of partition walls, and
the sealing step is performed during a period from an end of the filling step to an end of the ultraviolet light irradiation step, or the ultraviolet light irradiation step and the sealing step are performed under an identical sterile environment.

2. (Currently amended) The ultraviolet sterilization method according to claim 1, wherein
the sealing step is performed by heat-sealing of an aperture of the flexible bag.

3. (Original) The ultraviolet sterilization method according to claim 1 or 2, wherein
in the ultraviolet light irradiation step, "a maximum length at which an optical axis of the ultraviolet light applied crosses a container" or "intensity of ultraviolet light to be applied" in the ultraviolet transmissive container is controlled such that irradiance of the ultraviolet light at an arbitrary position of the object to be sterilized, which is filled into the container, is set to 0.01 mW/cm$^2$ or more.

4. (Canceled)

5. (Currently amended) The ultraviolet sterilization method according to any one of claims 1 to 3, wherein
in the ultraviolet light irradiation step, the ultraviolet transmissive container filled with the object to be sterilized is disposed such that 50% or more of an area of the surface of the ultraviolet transmissive container comes close to or abuts on the partition wall, and is irradiated with ultraviolet light from the two ultraviolet light-emitting surfaces respectively provided to both of the two facing surfaces of the pair of partition walls.

6. (Original) The ultraviolet sterilization method according to claim 5, wherein
the ultraviolet light-emitting surface is formed into a shape corresponding to a shape of the surface of the ultraviolet transmissive container filled with the object to be sterilized, and ultraviolet irradiation is performed such that 80% or more of an area of the surface of the ultraviolet transmissive container comes close to or abuts on the partition wall.

7. (Canceled)

8. (Currently amended) The ultraviolet sterilization method according to any one of claims 1 to [[7]]3, 5, and 6, further comprising
an ultraviolet blocking step of performing ultraviolet blocking treatment on an outer surface of the ultraviolet transmissive container sealed by the sealing step.

9. (Currently amended) A method of manufacturing a container-packaged product in which food, a cosmetic, a quasi-drug, or a drug, which is subjected to ultraviolet sterilization, is sealed in a container, the method comprising
a step of filling a container with a fluid object to be sterilized including food, a cosmetic, a quasi-drug, or a drug, performing ultraviolet sterilization, and sealing the container by the ultraviolet sterilization method according to any one of claims 1 to [[8]]3, 5, 6, and 8.

10. (Original) An ultraviolet sterilization apparatus, comprising:

a pair of partition walls that each include an ultraviolet transmissive window and are configured to be capable of pinching an ultraviolet transmissive container with the ultraviolet transmissive windows, the ultraviolet transmissive container being filled with an object to be sterilized; and
ultraviolet light sources that are each provided to the inside of the pair of partition walls and irradiate the ultraviolet transmissive container with ultraviolet light via the ultraviolet transmissive windows.

11. (Original) The ultraviolet sterilization apparatus according to claim 10, wherein
the ultraviolet transmissive window has a shape corresponding to a shape of the pinched ultraviolet transmissive container.

12. (Original) The ultraviolet sterilization apparatus according to claim 10 or 11, wherein
the ultraviolet light source includes a plurality of linear or point light sources disposed along the light-emitting surface.

13. (Original) The ultraviolet sterilization apparatus according to any one of claims 10 to 12, wherein
the pair of partition walls each include an aperture closed by the ultraviolet transmissive window, and the ultraviolet light source is configured to be movable inside the aperture along the ultraviolet transmissive window.

FIG.1

116    112
115    114
X    X´
113    111

111    113

X    X´

112

116

117

110

118

FIG.2

FIG.3

FIG.4

**402**

FIG.5

<u>403</u>

FIG.6

FIG.7

FIG.8

**INTERNATIONAL SEARCH REPORT**

| International application No. |
| --- |
| PCT/JP2016/064437 |

| A. CLASSIFICATION OF SUBJECT MATTER |
| --- |
| *B65B55/16*(2006.01)i, *A23L3/28*(2006.01)i, *A61L2/10*(2006.01)i |

According to International Patent Classification (IPC) or to both national classification and IPC

| B. FIELDS SEARCHED |
| --- |

Minimum documentation searched (classification system followed by classification symbols)
B65B55/16, A23L3/28, A61L2/10

Documentation searched other than minimum documentation to the extent that such documents are included in the fields searched
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>Y<br>A | JP 2000-511497 A (Purepulse Technologies, Inc.),<br>05 September 2000 (05.09.2000),<br>page 13, line 6 to page 36, line 15; fig. 1 to 12<br>& US 5786598 A<br>column 5, line 35 to column 12, line 14; fig. 1 to 8<br>& US 5925885 A          & US 6433344 B1<br>& US 6566659 B1          & US 2003/0155531 A1<br>& WO 1997/043915 A1      & EP 1413318 A1<br>& DE 69726425 T2         & AU 3000797 A<br>& CA 2254977 A1          & AU 710860 B2 | 1-6,9<br>8<br>7,10-13 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>12 August 2016 (12.08.16) | Date of mailing of the international search report<br>23 August 2016 (23.08.16) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2016/064437

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-068202 A (Toyo Seikan Group Holdings, Ltd.), 08 March 2002 (08.03.2002), paragraphs [0011] to [0013]; fig. 1 (Family: none) | 8 |
| A | JP 2005-52294 A (Iwasaki Electric Co., Ltd.), 03 March 2005 (03.03.2005), (Family: none) | 1-13 |
| A | GB 2457057 A (GB ENVIRONMENTAL LTD.), 05 August 2009 (05.08.2009), (Family: none) | 1-13 |
| A | JP 2014-516013 A (Ozonica Ltd.), 07 July 2014 (07.07.2014), & US 2012/0288405 A1 & US 2014/0119991 A1 & GB 2490794 A & WO 2012/153134 A2 & AU 2012252164 A1 & CA 2835562 A1 & CN 103619712 A & MX 2013013107 A & RU 2013154490 A & NZ 618094 A & ZA 201308984 B | 1-13 |
| A | JP 2007-126189 A (Iwasaki Electric Co., Ltd.), 24 May 2007 (24.05.2007), (Family: none) | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010058607 A
- JP 5566801 B
- JP 2012115715 A
- JP 2014087544 A
- JP 2006237563 A
- JP 2013534874 A
- JP 5591305 B

**Non-patent literature cited in the description**

- **ETSUZO MATSUI ; YOSHIHIRO SHIMIZU.** Ultraviolet Transmittance of Plastic Film II. *Report of Toyo Junior College of Food Technology and Toyo Institute of Food Technology,* 1967, 102-111